(19) Europäisches Patentamt
European Patent Office
Office européen des brevets

(11) **EP 3 593 709 B1**

(12) **FASCICULE DE BREVET EUROPEEN**

(45) Date de publication et mention de la délivrance du brevet:
**09.10.2024 Bulletin 2024/41**

(21) Numéro de dépôt: **19185888.5**

(22) Date de dépôt: **11.07.2019**

(51) Classification Internationale des Brevets (IPC):
**A61B 5/00** *(2006.01)* **A61B 5/16** *(2006.01)*

(52) Classification Coopérative des Brevets (CPC):
**A61B 5/7267; A61B 5/165;** A61B 5/681;
A61B 5/6824; G16H 50/70

(54) **PROCÉDÉ DE DÉTERMINATION DE L'ÉTAT DE STRESS D'UN INDIVIDU**

VERFAHREN ZUR BESTIMMUNG DES STRESSZUSTANDS EINER PERSON

METHOD FOR DETERMINING THE STATE OF STRESS OF AN INDIVIDUAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.07.2018 FR 1856504**

(43) Date de publication de la demande:
**15.01.2020 Bulletin 2020/03**

(73) Titulaires:
• **Commissariat à l'Énergie Atomique et aux Énergies Alternatives**
  **75015 Paris cedex (FR)**
• **Université Grenoble Alpes**
  **38400 Saint-Martin-d'Hères (FR)**

(72) Inventeurs:
• **VILA, Gaël**
  **38054 GRENOBLE cedex 09 (FR)**
• **GODIN, Christelle**
  **38054 GRENOBLE cedex 09 (FR)**
• **CAMPAGNE, Aurélie**
  **38600 FONTAINE (FR)**
• **CHARBONNIER, Sylvie**
  **38130 ECHIROLLES (FR)**

(74) Mandataire: **INNOV-GROUP**
**310, avenue Berthelot**
**69372 Lyon Cedex 08 (FR)**

(56) Documents cités:
**US-A1- 2013 137 996**

• **"Recent Application in Biometrics", 27 July 2011, INTECH, ISBN: 978-953-30-7488-7, article ALBERTO DE ET AL: "Real-Time Stress Detection by Means of Physiological Signals", XP055574626, DOI: 10.5772/18246**
• **KREINOVICH VLADIK ET AL: "Simple linear interpolation explains all usual choices in fuzzy techniques: Membership functions, t-norms, t-conorms, and defuzzification", 2015 ANNUAL CONFERENCE OF THE NORTH AMERICAN FUZZY INFORMATION PROCESSING SOCIETY (NAFIPS) HELD JOINTLY WITH 2015 5TH WORLD CONFERENCE ON SOFT COMPUTING (WCONSC), IEEE, 17 August 2015 (2015-08-17), pages 1 - 5, XP033219046, DOI: 10.1109/NAFIPS-WCONSC.2015.7284162**

**Description**

## DOMAINE TECHNIQUE

Le domaine technique de l'invention est la détermination d'un niveau de stress d'un individu à partir d'au moins une mesure d'un paramètre physiologique effectuée sur l'individu. La détermination du niveau de stress est établie en mettant en oeuvre une fonction d'appartenance, découlant de la logique floue.

## ART ANTERIEUR

Il est possible de déterminer un niveau de stress d'un individu à partir de mesures d'un ou de plusieurs paramètres physiologiques de ce dernier. Le paramètre physiologique peut être une activité cardiaque, mesurée par exemple par un électrocardiogramme (ECG) ou une simple détermination d'une fréquence cardiaque, ou une activité musculaire, mesurée par un électromyogramme (EMG), ou encore une mesure de la conductance électrique de la peau. La publication Ollander "A comparison of wearable and stationary sensors for stress détection", 2016 IEEE international conférence on Systems, Man and Cybernetics (SMC), Oct 2016, décrit comment certains paramètres physiologiques peuvent être utilisés pour établir un indicateur de stress d'un individu.

L'émergence de capteurs nomades et connectés, destinés à être portés par un individu, permet d'accéder à des mesures de paramètres physiologiques de façon simple et peu coûteuse. Il s'agit par exemple de capteurs spécifiques pouvant être fixés à un bracelet ou intégrés dans des montres ou reliés à des smartphones. Par exemple, le dispositif Empatica E4 comporte différents capteurs permettant d'accéder aisément à des paramètres physiologiques de type activité électrodermale, activité cardiaque ou température.

A partir des paramètres mesurés, des algorithmes de classification peuvent être mis en oeuvre, de façon à déterminer si l'individu se trouve dans un état de stress ou dans un état de repos. Certains algorithmes de classification sont basés sur la logique floue. Ce type d'algorithme est par exemple décrit dans la publication Kumar M "Fuzzy techniques for subjective workload-score modeling under uncertainties", IEEE transactions on systems, man and cybernetics- part B, Vol. 38, No. 6, December 2008. Ce type d'algorithme suppose l'établissement d'une phase d'apprentissage, au cours de laquelle un individu est placé dans une situation de stress, ou dans diverses situations de stress. La publication De Santos Sierra A. et Al "Real-time stress détection by means of physiological signals", Recent Application in Biometrics (2011-07-27) décrit une approche analogue, selon laquelle au cours de l'apprentissage, l'utilisateur doit être successivement dans un état de stress ainsi que dans un état de repos. Le fait d'imposer un apprentissage, durant lequel l'individu est placé dans une situation de stress, est contraignant. De plus, la fiabilité de telles méthodes peut être compromise par les variabilités physiologiques d'un individu à un autre.

Les inventeurs proposent d'établir un procédé de détermination d'un niveau de stress d'un individu ne nécessitant pas de placer l'individu dans un état de stress lors de la calibration. Cela permet une mise en oeuvre particulièrement simple du procédé, à partir d'équipements portés par l'individu. De plus, la calibration peut être renouvelée de façon périodique.

## EXPOSE DE L'INVENTION

Un premier objet de l'invention est un procédé de détermination d'un niveau de stress d'un individu, en fonction d'un paramètre physiologique de l'individu, dont la valeur est susceptible de varier en fonction du niveau de stress de l'individu, le procédé comportant les étapes suivantes :

a) mesure d'une valeur du paramètre physiologique en différentes périodes de calibration;
b) définition d'une plage de variation des valeurs du paramètre physiologique mesurées lors des différentes périodes de calibration ;
c) en fonction de la plage de variation, établissement d'une fonction d'appartenance, déterminant un niveau de stress de l'individu en fonction de la valeur du paramètre physiologique, le niveau de stress déterminé par la fonction d'appartenance évoluant ou pouvant évoluer entre :

- un niveau de repos, correspondant à un état de repos de l'individu, l'état de repos correspondant de préférence à l'état dans lequel se trouve l'individu lors des périodes de calibration ;
- un niveau de stress, correspondant à un état de stress de l'individu ;
- et au moins un niveau intermédiaire, compris entre le niveau de repos et le niveau de stress ;

d) suite à l'étape c), mesure d'une valeur du paramètre physiologique durant une période de mesure, et détermination du niveau de stress de l'individu, durant la période de mesure, en appliquant la fonction d'appartenance à la valeur du paramètre physiologique mesurée durant la période de mesure.

Le procédé peut être tel que :

- lors de l'étape c), le niveau de stress et chaque niveau intermédiaire sont déterminés en fonction de valeurs des paramètres physiologiques mesurées lors des périodes de calibration, l'individu étant de préférence dans un état de repos.
- lors de l'étape c), le niveau de stress et chaque niveau intermédiaire sont déterminés en fonction d'une distance entre la valeur du paramètre physiologique mesurée, lors de l'étape d), c'est-à-dire lors de la période de mesure, et la plage de variation établie lors de l'étape b).
- lors de l'étape a), lors de chaque période de calibration, l'individu est dans un état de repos ou est considéré comme étant dans un état de repos ;

L'étape c) peut comporter une prise en compte d'une distance seuil, de telle sorte que :

- le niveau de repos correspond à une valeur du paramètre physiologique comprise dans la plage de variation résultant de l'étape b) ;
- le niveau de stress correspond à une valeur du paramètre physiologique dont une distance, par rapport à la plage de variation, est supérieure à la distance seuil;
- chaque niveau intermédiaire correspond à une valeur du paramètre physiologique en dehors de la plage de variation, et dont la distance, par rapport à la plage de variation, est inférieure à la distance seuil.

L'étape c) peut comporter un calcul d'un indicateur de dispersion des valeurs du paramètre physiologique mesurées lors des différentes périodes de calibration, la distance seuil étant déterminée en fonction de l'indicateur de dispersion. L'indicateur de dispersion peut être ou comprendre une étendue de la plage de variation, correspondant à un écart entre une valeur minimale et une valeur maximale de la plage de variation. La distance seuil peut être obtenue en appliquant un facteur d'échelle à l'étendue de la plage de variation.

L'étape c) peut comporter une attribution d'une valeur représentative de la plage de variation en fonction de quoi :

- l'état de stress correspond à une valeur du paramètre physiologique dont la distance, par rapport à la valeur représentative, est supérieure à la distance seuil ;
- chaque niveau intermédiaire correspond à une valeur du paramètre physiologique dont la distance, par rapport à la valeur représentative, est inférieure à la distance seuil.

Le paramètre physiologique peut être ou peut comporter :

- une caractéristique de l'activité cardiaque de l'individu ;
- ou une caractéristique d'une activité musculaire de l'individu ;
- ou une caractéristique de l'activité corticale de l'individu ;
- ou une caractéristique de l'activité électrodermale de l'individu ;
- ou une température corporelle de l'individu ;
- ou une caractéristique représentative d'un mouvement de l'individu.

Selon un mode de réalisation :

- les étapes a) à c) sont mises en oeuvre indépendamment les unes des autres, respectivement pour différents paramètres physiologiques, pour établir autant de fonctions d'appartenance que de paramètres physiologiques considérés, chaque fonction d'appartenance étant associée à un paramètre physiologique ;
- l'étape d) comporte une mesure des différents paramètres physiologiques, durant la période de mesure, et une détermination d'un niveau de stress de l'individu relativement à chaque paramètre physiologique, à l'aide de chaque fonction d'appartenance respectivement associée à chaque paramètre physiologique.

Le procédé peut comporter une étape e) de combinaison des niveaux de stress déterminés relativement à chaque paramètre physiologique, lors de l'étape d), pour obtenir un index multiparamétrique de niveau de stress. La combinaison peut être ou comprendre une somme, ou une somme pondérée. Lors de l'étape e), l'index multiparamétrique de niveau de stress peut être déterminé par un calcul d'une moyenne pondérée ou d'une médiane des niveaux de stress respectivement déterminés relativement à chaque paramètre physiologique.

Selon ce mode de réalisation, lors de l'étape c), chaque fonction d'appartenance peut être définie indépendamment l'une de l'autre.

Selon ce mode de réalisation, à chaque paramètre physiologique peuvent être associées une distance seuil ainsi qu'une plage de variation des valeurs du paramètres physiologiques mesurées durant les périodes de calibration.

Selon un mode de réalisation, la fonction d'appartenance, ou chaque fonction d'appartenance, est une fonction définie dans un intervalle compris entre la plage de variation et la plage de variation augmentée de la distance seuil. Elle peut être continue dans cet intervalle.

Un autre objet de l'invention est un dispositif de détermination d'un niveau de stress d'un individu, comportant :

- un capteur, configuré pour mesurer un paramètre physiologique de l'individu, le capteur mesurant une valeur du paramètre susceptible de varier en fonction du niveau de stress de l'individu ;
- un processeur, configuré pour mettre en oeuvre les étapes b) à c) d'un procédé selon le premier objet de l'invention, à partir de mesures de différentes valeurs du paramètre physiologique mesurées par le capteur lors de périodes de calibration, le processeur étant également configuré pour mettre en oeuvre l'étape d) à partir d'au moins une valeur du paramètre physiologique mesurée durant une période de mesure.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

## FIGURES

La figure 1 représente un dispositif permettant une mise en oeuvre de l'invention.
La figure 2 illustre les principales étapes d'un mode de réalisation de l'invention.
Les figures 3A, 3B et 3C montrent différents exemples d'une fonction d'appartenance.
La figure 4 illustre les principales étapes d'un autre mode de réalisation de l'invention.
Les figures 5A et 5B montrent des résultats expérimentaux, au cours desquels on a déterminé un indicateur de stress multi-paramètrique, en mettant en oeuvre l'invention.

## EXPOSE DE MODES DE REALISATION PARTICULIERS

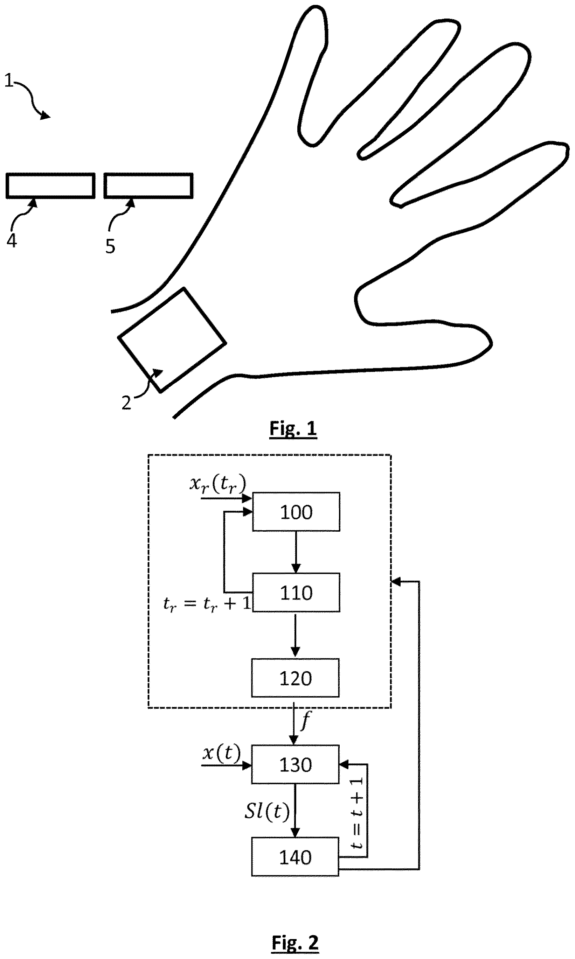

On a représenté, sur la figure 1, un dispositif 1 permettant la mise en oeuvre de l'invention. Un capteur 2 est disposé contre le corps d'un individu, par exemple au niveau du poignet. Le capteur 2 est configuré pour mesurer la valeur d'un paramètre physiologique $x(t)$ de l'individu durant une période de mesure $t$. Le terme période désigne un intervalle temporel, par exemple quelques secondes ou quelques minutes. La valeur du paramètre physiologique considéré peut varier en fonction d'un niveau de stress de l'individu. Le paramètre physiologique $x$ peut être un paramètre tel que décrit dans les publications citées dans l'art antérieur, en étant désigné par le terme "caratéristique" ou le terme anglais "feature". Le paramètre physiologique peut être par exemple :

- un paramètre lié à une activité cardiaque de l'individu, par exemple la fréquence cardiaque. Un tel paramètre peut être mesuré par des électrodes similaires à des électrodes d'un électrocardiogramme (ECG), ou par des moyens optiques, de type photoplethysmographie (PPG).
Il peut également s'agir d'une moyenne de la fréquence cardiaque (HR) ou d'un paramètre de dispersion de la fréquence cardiaque tel que l'écart-type, calculés durant une période temporelle prédéterminée. Le paramètre physiologique peut résulter d'une analyse fréquentielle de l'activité cardiaque dont celle de l'intervalle inter-battements, connu de l'homme du métier sous la désignation IBI (Inter Beat Interval). Sur un ECG, cet intervalle peut s'étendre entre deux pics R de deux impulsions cardiaques consécutives. L'analyse fréquentielle peut être effectuée dans des plages de fréquences comprises entre 0 Hz et 0.5 Hz, et plus spécifiquement, par exemple entre 0 et 0.003 Hz (ultra basses fréquences), ou entre 0.003 et 0.04 Hz (très basses fréquences), entre 0.04 Hz et 0.15 Hz (basses fréquences), ou entre 0.15 Hz et 0.5 Hz (hautes fréquences).

- Un paramètre lié à une activité musculaire de l'individu, ce type de paramètre pouvant être mesuré par électromyographie (EMG).

- Un paramètre lié à l'activité électrodermale de l'individu, représentatif de propriétés de conduction électrique ou d'impédance électrique de la peau (par exemple la conductance ou l'impédance électrodermale) et/ou de propriétés capacitives de la peau. Il peut s'agir d'une valeur moyenne de la conductance de la peau ou de la moyenne de valeur absolue d'une dérivée de la conductance de la peau. Il peut également s'agir d'une fréquence, ou d'une durée moyenne ou d'une amplitude moyenne des réponses électrodermales calculées durant une période temporelle prédéterminée. Plus généralement, le paramètre considéré est une caractéristique d'une réponse électroderma le.

- Une température de la peau de l'individu.

- Une mesure d'un mouvement, par exemple d'une accélération selon au moins un axe, une telle mesure permettant de caractériser un tremblement ou une activité physique susceptible d'impacter la physiologie de l'individu. Le paramètre considéré peut être une norme de l'accélération.

- Une mesure d'une activité corticale, par exemple à partir d'électrodes de type électroencéphalographie (EEG). Il peut par exemple s'agir d'une puissance spectrale dans une bande de fréquence prédéterminée calculée sur une période temporelle prédéterminée.

Le paramètre physiologique mesuré peut être un paramètre représentatif de l'activité cardiaque. Si $HR_j$ correspond à la fréquence cardiaque mesurée en un instant $j$, le paramètre physiologique $x(t)$ à l'instant $t$ peut être tel que :

$$x(t) = \sqrt{\frac{1}{N_j} \sum_j \left( HR_j - HR_{j-1} \right)^2}$$

, où $N_j$ correspond aux nombres de mesures de fréquence cardiaque prises en compte, avec : $t - N_j \leq j <\_ t$. $N_j$ est dimensionné pour prendre en compte les mesures de fréquence cardiaque durant une durée glissante de quelques secondes ou quelques dizaines de secondes, voire quelques minutes, par exemple 60s.

Si $IBI_j$ correspond à l'intervalle inter-battements mesuré en un instant $j$, le paramètre physiologique $x(t)$ à l'instant $t$ peut être tel que :

$$x(t) = \sqrt{\frac{1}{N_j} \sum_j \left( IBI_j - IBI_{j-1} \right)^2}$$

, où $N_j$ correspond aux nombres de mesures de fréquence cardiaque prises en compte, avec : $t - N_j \leq j \leq t$. $N_j$ est dimensionné pour prendre en compte les mesures de fréquence cardiaque durant une durée glissante de quelques secondes, ou quelques dizaines de secondes, voire quelques minutes, par exemple 60s.

Les paramètres physiologiques décrits dans les deux paragraphes qui précèdent conviennent particulièrement bien à une mise en oeuvre de l'invention.

L'objectif de l'invention est de déterminer un niveau de stress $SI(t)$ de l'individu à différentes périodes de mesure $t$.

Le capteur 2 est relié à un microprocesseur 4, ce dernier étant relié à une mémoire 5 dans laquelle sont stockées des instructions pour mettre en oeuvre le procédé décrit ci-après. Le microprocesseur 4 reçoit les mesures du capteur 2, par une liaison filaire ou une liaison sans fil. Le microprocesseur 4 peut être porté par l'individu, en étant disposé au niveau du capteur ou en étant implanté dans un dispositif annexe porté par la personne, par exemple un objet nomade tel un smartphone. Le microprocesseur 4 peut également être distant de l'individu.

La figure 2 décrit les principales étapes d'un premier mode de réalisation de l'invention. Le procédé suppose une phase de calibration, ou d'apprentissage, au cours de laquelle on effectue un paramétrage du procédé. Il s'agit d'établir une fonction d'appartenance $f$, qui permet de relier la valeur du paramètre mesurée à un niveau de stress. Plus précisément, et comme décrit par la suite, la fonction d'appartenance $f$ permet de relier la valeur mesurée du paramètre à un niveau d'appartenance à un état de stress ou à un état de repos.

La phase de calibration comprend les étapes 100 à 120. Un aspect important de l'invention est qu'au cours de cette phase, l'individu est au repos, ou, plus précisément, se considère comme étant au repos. Ainsi, la phase de calibration comporte des périodes de calibration $t_r$, au cours desquelles l'individu est considéré comme uniquement dans un état de repos. Il n'est donc pas dans un état de stress. Au cours de la phase de calibration, le capteur 2 mesure une valeur du paramètre physiologique $x_r(t_r)$, à différentes périodes $t_r$ l'indice $r$ désignant le fait que l'individu est considéré comme étant au repos. Si la calibration est effectuée alors que l'individu est dans un certain état de stress, cela nuit à la fiabilité de la détermination de l'état de stress de l'individu au cours d'instants de mesures postérieurs à la calibration.

Par état de repos d'un individu, on entend par exemple un état dans lequel l'individu est éveillé, mais son activité physique et mentale est minimale. Par exemple, l'individu est seul, assis ou allongé, et n'effectue aucune activité particulière. Dans la suite de la description, l'état de repos correspond à l'état dans lequel est l'individu durant la calibration.

Au cours de l'étape de mesure 100, le capteur mesure la valeur du paramètre physiologique $x_r(t_r)$ correspondant à la période de calibration $t_r$.

Au cours de l'étape 110, on incrémente la période de calibration $t_r$, puis on réitère l'étape 100, ou on sort de la boucle d'itérations formée par les étapes 100 et 110. Lors de l'étape 110, la sortie de la boucle d'itérations peut être effectuée au bout d'un nombre prédéterminé d'itérations, ou en fonction des valeurs $x_r(t_r)$ mesurées aux différentes périodes de calibration $t_r$. Par exemple, on peut calculer une grandeur statistique à partir des valeurs $x_r(t_r)$ mesurées

lors des périodes de calibration $t_r$ et arrêter les itérations en fonction d'une évolution de cette grandeur statistique, et en particulier lorsque l'évolution de la grandeur statistique est négligeable. La grandeur statistique peut être la moyenne, ou la médiane, ou un indicateur de dispersion de type variance, ou écart-type, ou un écart entre une valeur maximale $x_{r,max}$ et une valeur minimale $x_{r,min}$ du paramètre physiologique $x$.

Au cours de l'étape 120, on utilise les valeurs $x_r(t_r)$ mesurées au cours des différentes périodes de calibration $t_r$ pour définir la fonction d'appartenance $f$. Par exemple, on détermine une plage de variation $X_r$, dans laquelle s'étendent les valeurs $x_r(t_r)$ mesurées au cours des différentes périodes de calibration $t_r$, dites valeurs de calibration. La plage de variation $X_r$ est délimitée par une valeur minimale $x_{r,min}$ et une valeur maximale $x_{r,max}$. Ainsi, $X_r = [x_{r,min}, x_{r,max}]$. La plage de variation peut être caractérisée par son étendue $\Delta x_r$. L'étendue est telle que $\Delta x_r = x_{r,max} - x_{r,min}$ (1).

L'étape 120 peut comporter l'application d'un test statistique pour éliminer des valeurs $x_r(t_r)$ aberrantes. Un test de Dixon, connu de l'homme du métier, peut par exemple être effectué. L'élimination de valeurs aberrantes permet d'améliorer la fiabilité du procédé.

On peut également déterminer une distance seuil $d_S$. La distance seuil $d_S$ peut être telle que: $d_S = \alpha \times \Delta x_r$, (2), $\alpha$ étant un réel positif, désigné par le terme facteur d'échelle. La valeur du facteur d'échelle $\alpha$ dépend du paramètre physiologique considéré. Elle est typiquement comprise entre 0.1 et 0.5. Le facteur d'échelle permet de déterminer la distance seuil $d_S$ à partir de l'étendue $\Delta x_r$, comme décrit par la suite.

La fonction d'appartenance $f$ est destinée à définir un niveau de stress $Sl$ à partir de la valeur d'un paramètre physiologique $x(t)$ mesurée à une période de mesure $t$, postérieur à la phase de calibration. Le niveau de stress $Sl$ peut par exemple varier entre 0 et 1, 0 correspondant à un état de repos et 1 correspondant à un état de stress de l'individu. Selon les principes de la logique floue, la fonction d'appartenance $f$ peut définir des niveaux intermédiaires, compris entre 0 et 1, et correspondant à un état de stress intermédiaire. La fonction d'appartenance $f$ est de préférence continue dans un espace de départ $E$ défini par les valeurs susceptibles d'être prises par le paramètre physiologique mesuré.

L'espace de départ $E$ peut par exemple être l'ensemble des réels positifs. Ainsi, $$f : E = \mathbb{R}^+ \to [0,1]$$ et $f(x(t)) = Sl(t)$.

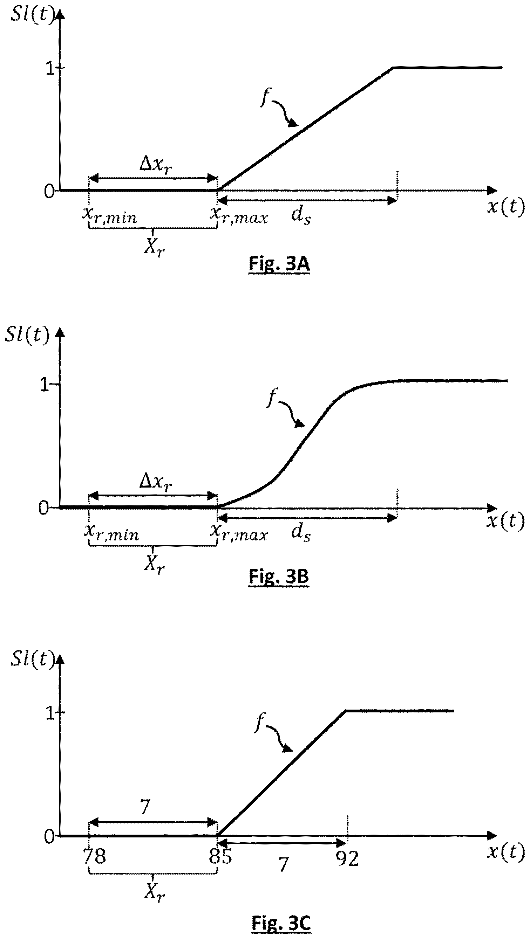

Un exemple de fonction d'appartenance $f$ est illustré sur la figure 3A :

- Lorsque $(t) \in X_r$, c'est-à-dire lorsque $x_{r,min} \le x(t) \le x_{r,max}$, $Sl(t) = f(x(t)) = 0$ (3). Dans ce cas, la valeur du paramètre mesurée $x(t)$ appartient à la plage de variation $X_r$. Le niveau de stress de l'individu est égal à 0, ce qui signifie que l'individu est considéré comme étant au repos, c'est-à-dire dans un même état que celui durant lequel la calibration a été effectuée. Il en est également ainsi lorsque $x(t) \le x_{r,min}$.

- Lorsque $(t) \ge x_{r,max} + d_S$, $Sl(t) = f(x(t)) = 1$ (4). Le niveau de stress de l'individu est égal à 1, ce qui signifie que l'individu est considéré comme étant dans un état de stress. Ainsi, lorsque la valeur mesurée $x(t)$ du paramètre est distante d'une distance supérieure à la distance seuil $d_S$, de la plage de variation $X_r$, l'individu est considéré comme étant dans un état de stress.

  D'une façon plus générale, la fonction d'appartenance $f$ est telle que lorsqu'une distance $d(x(t), X_r)$ entre la valeur du paramètre physiologique $x(t)$ et la plage de variation $X_r$, est supérieure à la distance seuil $d_S$, le niveau de stress $Sl(t)$ est égal à 1. Dans cet exemple, $d(x(t), X_r) = d(x(t), x_{r,max})$. En utilisant la définition de la distance seuil donnée dans l'expression (2), la condition (4) peut s'écrire:

$$\text{Si } (x(t), X_r) > \alpha \times \Delta x_r, Sl(t) = f(x(t)) = 1 \text{ (5).}$$

  La distance $d(x(t), X_r)$ peut être une distance euclidienne ou une autre distance.

- Lorsque $x_{r,max} < x(t) < x_{r,max} + d_S$, $Sl(t) = f(x(t)) \in\ ]0,1[$ (6). Le niveau de stress de l'individu est un niveau intermédiaire, compris entre 0 et 1. Dans l'exemple représenté sur la figure 3A, la fonction d'appartenance est linéaire par morceaux.

  Aussi, dans cet exemple, lorsque $x_{r,max} \le x(t) \le x_{r,max} + d_S$, $Sl(t) = f(x(t)) = \dfrac{x(t) - x_{r,max}}{d_S}$. La normalisation par $d_S$ permet d'obtenir des niveaux d'état intermédiaire compris entre 0 (lorsque $x(t) = x_{r,max}$) et 1 (lorsque $x(t) = x_{r,max} + d_S$). Ainsi, lorsque la valeur mesurée est comprise entre $x_{r,max}$ et $x_{r,max} + d_S$, la valeur de la fonction d'appartenance varie de façon monotone entre 0 et 1. Dans cet exemple, la fonction d'appartenance est croissante. Plus $x(t)$ se rapproche de $x_{r,max} + d_S$, plus la valeur de la fonction d'appartenance est élevée.

  D'une façon plus générale, la fonction d'appartenance $f$ est telle que : lorsque $x(t) \notin X_r$ et $d(x(t), X_r) < d_S$, (7) le niveau de stress est un niveau intermédiaire, compris entre le niveau correspondant à l'état de repos (c'est-à-dire l'état dans lequel la calibration a été effectuée) et le niveau correspondant à l'état de stress. Lorsque les niveaux de repos

$$Sl(t) = \frac{d(x(t), X_r)}{d_S} \quad (8)$$

et de stress sont respectivement fixés à 0 et 1, et que la fonction d'appartenance est linéaire, .

Selon une variante, représentée sur la figure 3B, la fonction *f* n'est pas linéaire par morceaux . Elle peut par exemple prendre une forme de type tangente hyperbolique ou de toute autre fonction sigmoïde.

L'exemple donné ci-dessus est valable lorsque la fonction d'appartenance *f* est croissante, c'est-à-dire que le niveau de stress est d'autant plus élevé que la valeur mesurée du paramètre physiologique l'est également. Dans certains cas de figure, par exemple lorsque le paramètre considéré est la résistance de la peau, la fonction d'appartenance *f* est décroissante : le niveau de stress est d'autant plus élevé que la valeur du paramètre mesurée est faible. Dans une telle configuration :

- lorsque $x_{r,min} \leq x(t)$, $Sl(t) = f(x(t)) = 0$ ;

- lorsque $(t) \leq x_{r,min} - d_S$, $Sl(t) = f(x(t)) = 1$ (9) ;

- lorsque $x_{r,min} - d_S < x(t) < x_{r,min}$, $Sl(t) = f(x(t)) \in \, ]0,1[$ (10). Si la fonction d'appartenance est linéaire par morceaux,

$$Sl(t) = f(x(t)) = \frac{x_{r,min} - x(t)}{d_S}$$

alors lorsque $x_{r,min} - d_S < x(t) < x_{r,min}$, . La normalisation par $d_S$ permet d'obtenir des niveaux d'état intermédiaire compris entre 0 ($x(t) \to x_{r,min}$) et 1 ($x(t) \to x_{r,min} - d_S$). La flèche $\to$ signifie "tend vers".

D'une façon générale, on peut attribuer une valeur représentative à la plage de variation $X_r$. En fonction de la distance *d* entre la valeur du paramètre $x(t)$ et la valeur représentative, la fonction d'appartenance *f* détermine un niveau de stress $Sl(t)$. Dans les exemples donnés ci-dessus, on a respectivement pris, comme valeur représentative, la valeur maximale $x_{r,max}$ et la valeur minimale $x_{r,min}$ de la plage de variation. Selon d'autres exemples, la valeur représentative peut être un indicateur statistique appliqué aux valeurs de calibration $x_r(t_r)$ mesurées au cours de la calibration. Il peut par exemple s'agir de la moyenne $\overline{X_r}$ ou de la médiane med($X_r$) des valeurs de calibration. Il peut également s'agir d'un fractile, par exemple un quartile (le premier quartile, lorsque la fonction d'appartenance est décroissante ou le quatrième quartile lorsque la fonction d'appartenance est croissante) ou un décile (par exemple le premier décile lorsque la fonction d'appartenance est décroissante ou le dixième décile lorsque la fonction d'appartenance est croissante). Le niveau de stress, correspondant à une valeur mesurée d'un paramètre $x(t)$, peut alors être calculé en fonction de la distance entre la valeur du paramètre et la valeur représentative de la plage de variation. La distance *d* peut être normalisée par un indicateur de la dispersion des valeurs de calibration, par exemple l'étendue $\Delta x_r$ de la plage de variation ou l'écart type des valeurs de calibration $x_r(t_r)$.

Le facteur d'échelle $\alpha$ peut être déterminé en fonction d'un indicateur de dispersion des valeurs mesurées au cours de la calibration. L'indicateur de dispersion peut être l'étendue $\Delta x_r$ de la plage de variation $X_r$. Il peut également s'agir d'une variance ou d'un écart type des valeurs de calibration $x_r(t_r)$.

Les étapes 130 et 140, décrites ci-après, correspondent à une phase d'utilisation du capteur 2 pour estimer un état de stress de l'individu pour lequel la fonction d'appartenance *f* a été définie. Au cours de l'étape 130, on effectue une mesure du paramètre physiologique $x(t)$ à une période de mesure *t*. Le paramètre physiologique $x(t)$ mesuré à chaque période de mesure est le même que celui mesuré lors des périodes de calibration.

Au cours de l'étape 140, on applique la fonction d'appartenance *f,* préalablement définie, à la valeur $x(t)$ du paramètre physiologique à la période de mesure, de façon à déterminer un niveau de stress $Sl(t) = f(x(t))$. A l'issue de l'étape 140, la période peut être incrémentée et une autre itération des étapes 130 à 140 est effectuée.

Ainsi, selon le procédé précédemment décrit, la calibration n'est effectuée uniquement avec des valeurs du paramètre mesurées lors de la calibration, alors que l'individu est considéré comme étant dans un état de repos. La calibration ne nécessite pas d'effectuer des mesures du paramètre alors que l'individu se trouve dans un état de stress. Un avantage de la méthode est que la calibration est plus rapide et plus simple à réaliser. Un autre avantage est que la calibration peut être renouvelée périodiquement, afin de tenir compte d'une éventuelle variabilité physiologique de l'utilisateur. Dans ce cas, lorsqu'un renouvellement est souhaité, suite à l'étape 140, le procédé met en oeuvre les étapes 100 à 120. La calibration étant particulièrement simple à réaliser, des renouvellements de la calibration peuvent être réalisés fréquemment.

Un exemple d'application est représenté sur la figure 3C, le paramètre mesuré étant une fréquence cardiaque moyenne durant une période d'une minute. Au cours de la phase de calibration, les valeurs de la fréquence cardiaque moyenne ont été mesurées entre 78 et 85 battements par minutes. Ainsi, $\Delta x_r = 7$ . On obtient alors la fonction d'appartenance telle que représentée sur la figure 3C en considérant $\alpha = 1$. Ainsi :

- lorsque $x(t) < 85$, le niveau de stress est égal à 0, l'individu étant dans un état de repos ;
- lorsque $85 < x(t) \leq 92$, le niveau de stress est compris entre 0 et 1 ;
- lorsque $x(t) > 92$, le niveau de stress est égal à 1, l'individu étant dans un état de stress.

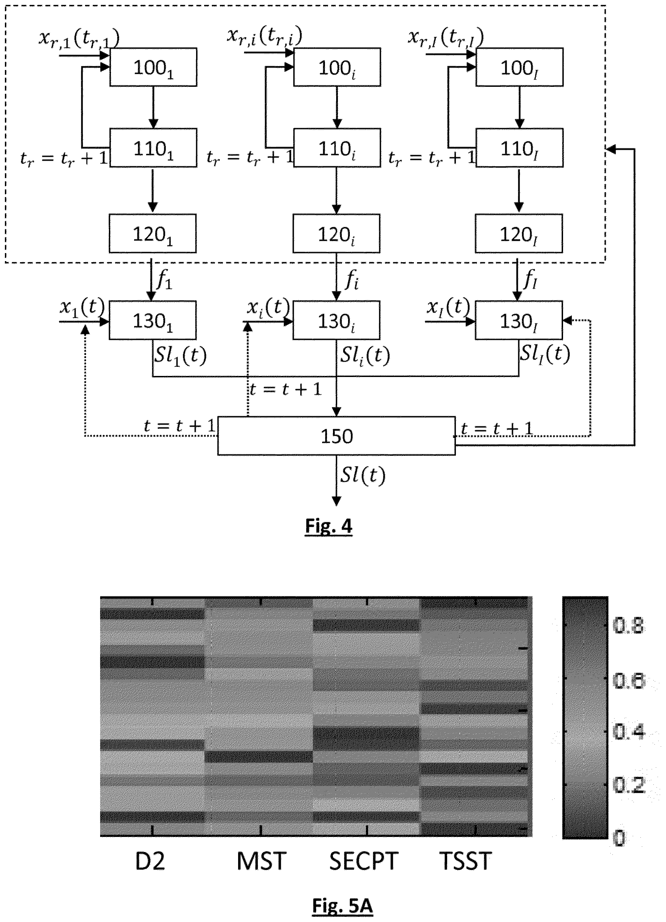

Selon un mode de réalisation, la méthode décrite ci-dessus peut être appliquée en mesurant simultanément différents paramètres $x_i$, l'indice i identifiant le paramètre considéré, avec $1 < i \leq I$, $I$ désignant le nombre de paramètres physiologiques considérés. On a représenté, sur la figure 4, les principales étapes de ce mode de réalisation.

Pour chaque paramètre physiologique $x_i$, on effectue une calibration selon des étapes $100_i$, $110_i$ et $120_i$. Ces étapes sont respectivement similaires aux étapes 100, 110 et 120 précédemment décrites. Elles sont mises en oeuvre respectivement à partir de valeurs $x_{r,1}(t_r)$ ... $x_{r,i}(t_r)$ ... $x_{r,I}(t_r)$, des paramètres physiologiques considérés, à différentes périodes de calibration $t_r$.

Chaque étape $120_i$ est effectuée en considérant une plage de variation $X_{r,i}$, du paramètre $x_i$ au cours de la calibration. La plage de variation $X_{r,i}$ présente une étendue $\Delta x_{r,i}$. A chaque paramètre physiologique $x_i$ est affecté un facteur d'échelle $\alpha_i$. On note que le facteur d'échelle $\alpha_i$ peut être différent d'un paramètre physiologique à un autre. L'étape $120_i$ permet de définir une fonction d'appartenance $f_i$ relativement au paramètre physiologique $x_i$. Les fonctions d'appartenance $f_i$, $f_{i+1}$ respectivement associées à de deux paramètres différents $x_i$, $x_{i+1}$, sont établies indépendamment l'une de l'autre. Il est cependant préférable que pour chaque fonction d'appartenance, l'état de repos et l'état de stress correspondent respectivement aux mêmes niveaux, par exemple 0 pour l'état de repos et 1 pour l'état de stress. On aboutit ainsi à une définition de $I$ fonctions d'appartenance $f_1$ ... $f_I$, respectivement associées aux $I$ paramètres physiologiques mesurés $x_1$ ... $x_I$. A chaque paramètre physiologique considéré peut correspondre une plage de variation, déterminée lors de la calibration, ainsi qu'une distance seuil. Chaque fonction d'appartenance $f_i$ est établie en fonction de la plage de variation et de la distance seuil associées à chaque paramètre physiologique.

Après que chaque fonction d'appartenance $f_i$ a été définie, le procédé comporte une étape $130_i$, mise en oeuvre pour chaque paramètre $x_i(t)$ mesuré à une période de mesure t, de façon à déterminer un niveau de stress $SI_i(t)$ associé à chaque paramètre $x_i(t)$, selon l'expression $SI_i(t) = f_i(x_i(t))$. On aboutit ainsi à une définition de $I$ niveaux de stress $SI_1(t) ... SI_I(t)$, respectivement associées aux $I$ paramètres physiologiques considérés $x_1$ ... $x_I$.

Au cours d'une étape 150, les différents niveaux de stress $SI_1(t) ... SI_I(t)$, respectivement associés à chaque paramètre $x_i(t)$, sont combinés, de façon à déterminer un niveau de stress global, ou multiparamétrique, $SI(t)$, selon les principes de la logique floue. La combinaison peut être un calcul d'une valeur moyenne ou d'une valeur médiane. Il peut également s'agir d'une moyenne pondérée, dans laquelle chaque niveau de stress $SI_i(t)$ est affecté d'un facteur de pondération $\lambda_i$ dépendant de l'importance que l'on souhaite attribuer au paramètre physiologique $x_i$ relativement aux autres paramètres considérés. La combinaison des différents niveaux de stress $SI_1(t) ... sl_I(t)$ peut être effectuée en appliquant des règles d'inférence prédéterminées.

A partir du niveau de stress multiparamétrique $SI(t)$, on peut déterminer si l'individu se trouve dans un état de stress. Des premiers essais expérimentaux ont montré que lorsque le niveau de stress multiparamétrique $SI(t)$ est supérieur à 0.3 ou 0.4, on peut considérer que l'individu se trouve dans un état de stress dans la période de mesure.

Par ailleurs, à partir du niveau de stress multiparamétrique $SI(t)$, on peut définir un état d'activité, correspondant à un état intermédiaire entre l'état de repos et l'état de stress. L'état d'activité correspond à un individu effectuant une activité physique ou mentale normale, sans être en état de stress. L'état d'activité correspond à un niveau de stress multiparamétrique s'étendant entre:

- le niveau de stress multiparamétrique pour lequel on considère que l'individu est en état de stress ;
- et niveau de stress multiparamétrique pour lequel on considère que l'individu est en état de repos.

Des essais expérimentaux ont été effectués sur une cohorte de 20 sujets âgés de 19 à 30 ans, soumis successivement à quatre situations de stress différentes:

- une première situation de stress, connue de l'homme du métier par le terme "test D2", au cours de laquelle le sujet doit reconnaître, en temps limité, des symboles distribués parmi des symboles visuellement proches, ces derniers étant désignés par le terme "distracteur". Il s'agit d'un test d'attention sélective usuel.
- Une deuxième situation de stress, connue de l'homme du métier par le terme "MST", signifiant "Mental Stress Test", au cours de laquelle le sujet doit compter, à rebours, en partant (par exemple) du nombre 1022, par incréments de 13, en étant filmé et en présence de deux observateurs.
- Une troisième situation de stress, connue de l'homme du métier par le terme "SECPT", signifiant "Socially Evaluated Cold-Pressure Test", au cours de laquelle le sujet place sa main dans de l'eau très froide (de 0° à 4°C) pendant trois minutes, en présence d'un observateur.

- Une quatrième situation de stress, connue de l'homme du métier par le terme "TSST", signifiant "Trier Social Stress Test", au cours de laquelle le sujet est filmé durant une simulation d'entretien de recrutement devant deux observateurs.

Au cours de chaque test, trois types de signaux physiologiques ont été mesurés :

- l'activité cardiaque a été mesurée en utilisant deux électrodes ECG ;
- la conductance cutanée a été mesurée grâce à deux électrodes situées sur la phalange médiane du majeur et de l'index de la main non dominante;
- l'activité musculaire a été mesurée en utilisant des électrodes EMG disposées au niveau du muscle corrugateur du sourcil et des muscles orbiculaires supérieurs et inférieurs.

Les signaux ont été acquis selon une fréquence d'acquisition de 1000 Hz.

Au cours d'une phase préliminaire, on a sélectionné des paramètres x, ou features, les plus adaptés à la détection d'une situation de stress. Il s'agit de :

- la moyenne de la fréquence cardiaque ;
- l'écart type de la fréquence cardiaque ;
- une puissance spectrale moyenne de l'intervalle inter-battement dans la bande spectrale des ultra-basses fréquences ;
- une puissance spectrale moyenne de l'intervalle inter-battement dans la bande spectrale des très basses fréquences ;
- la conductance cutanée moyenne ;
- la moyenne de la valeur absolue de la dérivée de la conductance cutanée;
- la proportion d'échantillons positifs dans la dérivée de la conductance cutanée.

La valeur de chaque paramètre est calculée en considérant une période de mesure comprise entre 3 et 5 minutes.

Pour chaque paramètre, on a établi une fonction d'appartenance, comme précédemment décrit.

On a ensuite combiné chaque paramètre, et sa fonction d'appartenance, pour former un indicateur de stress multiparamétrique, ou indicateur de stress "multi-features". Cet index est obtenu en effectuant une moyenne de la valeur de la fonction d'appartenance pour chaque paramètre.

La figure 5A représente, pour chaque situation de stress (axe des abscisses), et pour chaque individu testé (axe des ordonnées), une valeur de l'indicateur de stress multiparamétrique, représentée en niveaux de gris.

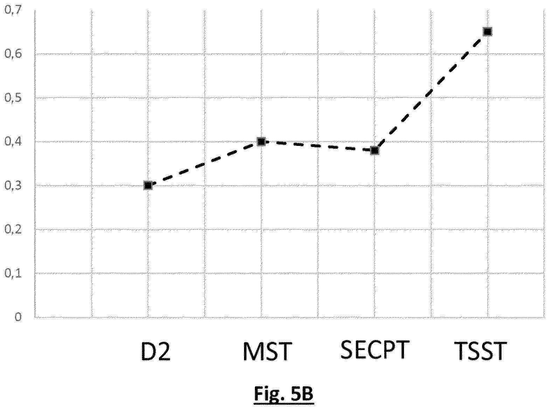

La figure 5B montre la valeur moyenne, pour l'ensemble des individus testés, de l'indicateur de stress multiparamétrique.

On observe que les valeurs les plus élevées de l'indicateur de stress sont obtenues pour le test TSST.

L'invention pourra être mise en oeuvre pour le suivi du niveau de stress d'individus. Il peut par exemple s'agir d'un suivi du niveau de stress en milieu professionnel, ou du suivi du niveau de stress chez des individus sujets à une anxiété lors de situations particulières, par exemple dans un moyen de transport. Elle peut également être appliquée au suivi du niveau de stress d'un sportif.

**Revendications**

1. Procédé de détermination d'un niveau de stress d'un individu, en fonction d'un paramètre physiologique de l'individu, dont la valeur est susceptible de varier en fonction du niveau de stress de l'individu, le procédé comportant les étapes suivantes :

   a) mesure d'une valeur du paramètre physiologique ($x_r(t_r)$), en différentes périodes de calibration ($t_r$) à l'aide d'un capteur;
   b) définition d'une plage de variation ($X_r$) des valeurs du paramètre physiologique mesurées lors des différentes périodes de calibration ;
   c) en fonction de la plage de variation ($X_r$), établissement d'une fonction d'appartenance ($f$), déterminant un niveau de stress ($SI$) de l'individu en fonction de la valeur du paramètre physiologique, le niveau de stress déterminé par la fonction d'appartenance évoluant entre :

- un niveau de repos, correspondant à un état de repos de l'individu ;
- un niveau de stress, correspondant à un état de stress de l'individu ;
- et au moins un niveau intermédiaire, compris entre le niveau de repos et le niveau de stress ;

d) suite à l'étape c), mesure d'une valeur du paramètre physiologique ($x(t)$) lors d'une période de mesure, et détermination du niveau de stress de l'individu ($Sl(t)$), durant la période de mesure, en appliquant la fonction d'appartenance ($f$) à la valeur du paramètre physiologique mesurée durant la période de mesure ;
le procédé étant tel que lors de l'étape c), le niveau de stress et chaque niveau intermédiaire sont déterminés :

- en fonction de valeurs des paramètres physiologiques mesurés, lors des périodes de calibration ;
- et en fonction d'une distance ($d(x(t),X_r)$) entre la valeur du paramètre physiologique mesurée lors de l'étape d) et la plage de variation ($X_r$) définie lors de l'étape b) ;

le procédé étant caractérisé en ce lors de l'étape a), lors de chaque période de calibration, l'individu est considéré comme étant dans un état de repos.

2. Procédé selon la revendication 1, dans lequel l'étape c) comporte une prise en compte d'une distance seuil ($d_S$), de telle sorte que :

- le niveau de repos correspond à une valeur du paramètre physiologique ($x(t)$) comprise dans la plage de variation ($X_r$) résultant de l'étape b) ;
- le niveau de stress correspond à une valeur du paramètre physiologique ($x(t)$) dont une distance ($d(x(t),X_r)$), par rapport à la plage de variation ($X_r$), est supérieure à la distance seuil ($d_S$);
- chaque niveau intermédiaire correspond à une valeur du paramètre physiologique ($x(t)$) en dehors de la plage de variation ($X_r$), et dont la distance ($d(x(t),X_r)$), par rapport à la plage de variation, est inférieure à la distance seuil ($d_S$).

3. Procédé selon la revendication 2, dans lequel l'étape c) comporte un calcul d'un indicateur de dispersion des valeurs ($x_r(t_r)$) du paramètre physiologique mesurées lors des différentes périodes de calibration, la distance seuil ($d_S$) étant déterminée en fonction de l'indicateur de dispersion.

4. Procédé selon la revendication 3, dans lequel l'indicateur de dispersion est ou comprend une étendue de la plage de variation ($\Delta x_r$), correspondant à un écart entre une valeur minimale ($x_{r,min}$) et une valeur maximale ($x_{r,max}$) de la plage de variation.

5. Procédé selon la revendication 4, dans lequel la distance seuil ($d_S$) est obtenue en appliquant un facteur d'échelle ($\alpha$) à l'étendue de la plage de variation.

6. Procédé selon l'une quelconque des revendications de 2 à 5, dans lequel l'étape c) comporte une attribution d'une valeur représentative de la plage de variation ($x_{r,max}$, $x_{r,min}$, $\overline{X}_r$) en fonction de quoi :

- l'état de stress correspond à une valeur du paramètre physiologique dont la distance, par rapport à la valeur représentative, est supérieure à la distance seuil ($d_S$) ;
- chaque niveau intermédiaire correspond à une valeur du paramètre physiologique dont la distance, par rapport à la valeur représentative, est inférieure à la distance seuil ($d_S$).

7. Procédé selon l'une des revendications précédentes, dans lequel le paramètre physiologique est ou comporte :

- une caractéristique de l'activité cardiaque de l'individu ;
- ou une caractéristique d'une activité musculaire de l'individu ;
- ou une caractéristique de l'activité corticale de l'individu ;
- ou une caractéristique de l'activité électrodermale de l'individu ;
- ou une température corporelle de l'individu ;
- ou une caractéristique représentative d'un mouvement de l'individu.

8. Procédé selon l'une quelconque des revendications précédentes, dans lequel :

- les étapes a) à c) sont mises en oeuvre indépendamment les unes des autres, respectivement pour différents paramètres physiologiques ($x_i$), pour établir autant de fonctions d'appartenance ($f_i$) que de paramètres physiologiques considérés, chaque fonction d'appartenance étant associée à un paramètre physiologique ;
- l'étape d) comporte une mesure des différents paramètres physiologiques ($x_i(t)$), durant la période de mesure, et une détermination d'un niveau de stress ($SI_i(t)$) de l'individu relativement à chaque paramètre physiologique, à l'aide de chaque fonction d'appartenance ($f_i$) respectivement associée à chaque paramètre physiologique ;

le procédé comporte une étape e) de combinaison des niveaux de stress ($SI_i(t)$) déterminés relativement à chaque paramètre physiologique ($x_i$), lors de l'étape d), pour obtenir un index multiparamétrique de niveau de stress ($SI(t)$).

9. Procédé selon la revendication 8, dans lequel lors de l'étape e), l'index multiparamétrique de niveau de stress est déterminé par un calcul d'une moyenne pondérée ou d'une médiane des niveaux de stress ($SI_i(t)$) respectivement déterminés relativement à chaque paramètre physiologique ($x_i$).

10. Procédé selon l'une des revendications 8 ou 9, dans lequel lors de l'étape c), chaque fonction d'appartenance ($f_i$) est définie indépendamment l'une de l'autre.

11. Procédé selon l'une quelconque des revendications 2 à 10, dans lequel la fonction d'appartenance, ou chaque fonction d'appartenance, est une fonction définie dans un intervalle compris entre la plage de variation et la plage de variation augmentée de la distance seuil, et continue dans cet intervalle.

12. Dispositif de détermination d'un niveau de stress d'un individu, comportant :

- un capteur (2), configuré pour mesurer un paramètre physiologique de l'individu, le capteur mesurant une valeur du paramètre susceptible de varier en fonction du niveau de stress de l'individu ;
- un processeur (4), configuré pour mettre en oeuvre les étapes b) à c) d'un procédé selon l'une des revendications précédentes, à partir de mesures de différentes valeurs du paramètre physiologique mesurées par le capteur lors de périodes de calibration, lors de lesquelles l'individu est considéré comme étant au repos, le processeur étant également configuré pour mettre en oeuvre l'étape d) à partir d'au moins une valeur du paramètre physiologique mesurée durant une période de mesure.


**Patentansprüche**

1. Verfahren zur Bestimmung eines Stresspegels einer Person in Abhängigkeit von einem physiologischen Parameter der Person, dessen Wert in Abhängigkeit von dem Stresspegel der Person variieren kann, wobei das Verfahren die folgenden Schritte umfasst:

a) Messen eines Werts des physiologischen Parameters ($x_r(t_r)$) in verschiedenen Kalibrierungsperioden ($t_r$) mithilfe eines Sensors;
b) Definieren eines Variationsbereichs ($X_r$) der Werte des physiologischen Parameters, die bei den verschiedenen Kalibrierungsperioden gemessen werden;
c) in Abhängigkeit von dem Variationsbereichs ($X_r$) Ermitteln einer Zugehörigkeitsfunktion ($f$), die einen Stresspegel ($SI$) der Person in Abhängigkeit von dem Wert des physiologischen Parameters bestimmt, wobei sich der durch die Zugehörigkeitsfunktion bestimmte Stresspegel ändert zwischen:

- einem Ruhepegel, der einem Ruhezustand der Person entspricht;
- einem Stresspegel, der einem Stresszustand der Person entspricht;
- und mindestens einem Zwischenpegel, der zwischen dem Ruhepegel und dem Stresspegel liegt;

d) nach dem Schritt c) Messen eines Werts des physiologischen Parameters ($x(t)$) bei einer Messperiode und Bestimmen des Stresspegels der Person ($SI(t)$) während der Messperiode, indem die Zugehörigkeitsfunktion ($f$) auf den Wert des physiologischen Parameters angewandt wird, der während der Messperiode gemessen wird; wobei das Verfahren derart ist, dass bei dem Schritt c) der Stresspegel und jeder Zwischenpegel bestimmt werden:

- in Abhängigkeit von Werten der physiologischen Parameter, die bei den Kalibrierungsperioden gemessen werden;

- und in Abhängigkeit von einem Abstand ($d(x(t), X_r)$) zwischen dem Wert des physiologischen Parameters, der bei dem Schritt d) gemessen wird, und dem Variationsbereich ($X_r$), der bei dem Schritt b) definiert wird;

wobei das Verfahren **dadurch gekennzeichnet ist, dass** die Person bei dem Schritt a) bei jeder Kalibrierungsperiode als in einem Ruhezustand befindlich betrachtet wird.

2. Verfahren nach Anspruch 1, wobei der Schritt c) eine Berücksichtigung eines Schwellenabstands ($d_s$) umfasst, so dass:

- der Ruhepegel einem Wert des physiologischen Parameters ($x(t)$) entspricht, der in dem aus dem Schritt b) hervorgegangenen Variationsbereich ($X_r$) enthalten ist;
- der Stresspegel einem Wert des physiologischen Parameters ($x(t)$) entspricht, bei dem ein Abstand ($d(x(t), X_r)$), in Bezug auf den Variationsbereich ($X_r$), größer als der Schwellenabstand ($d_s$) ist;
- jeder Zwischenpegel einem Wert des physiologischen Parameters ($x(t)$) entspricht, der außerhalb des Variationsbereichs ($X_r$) liegt und bei dem der Abstand ($d(x(t), X_r)$), in Bezug auf den Variationsbereich, kleiner als der Schwellenabstand ($d_s$) ist.

3. Verfahren nach Anspruch 2, wobei der Schritt c) eine Berechnung eines Streuungsindikators der Werte ($x_r(t_r)$) des physiologischen Parameters umfasst, die bei den verschiedenen Kalibrierungsperioden gemessen werden, wobei der Schwellenabstand ($d_s$) in Abhängigkeit von dem Streuungsindikator bestimmt wird.

4. Verfahren nach Anspruch 3, wobei der Streuungsindikator eine Größe des Variationsbereichs ($\Delta x_r$) ist oder aufweist, die einer Spanne zwischen einem kleinsten Wert ($x_{r,min}$) und einem größten Wert ($x_{r,max}$) des Variationsbereichs entspricht.

5. Verfahren nach Anspruch 4, wobei der Schwellenabstand ($d_s$) erhalten wird, indem ein Skalenfaktor ($\alpha$) auf die Größe des Variationsbereichs angewandt wird.

6. Verfahren nach einem der Ansprüche 2 bis 5, wobei der Schritt c) eine Zuweisung eines Werts umfasst, der für den Variationsbereich ($x_{r,max}$, $x_{r,min}$, $\overline{X}_r$) repräsentativ ist, in Abhängigkeit wovon:

- der Stresszustand einem Wert des physiologischen Parameters entspricht, dessen Abstand, in Bezug auf den repräsentativen Wert, größer als der Schwellenabstand ($d_s$) ist;
- jeder Zwischenpegel einem Wert des physiologischen Parameters entspricht, dessen Abstand, in Bezug auf den repräsentativen Wert, kleiner als der Schwellenabstand ($d_s$) ist.

7. Verfahren nach einem der vorhergehenden Ansprüche, wobei der physiologische Parameter ist oder umfasst:

- ein Merkmal der Herzaktivität der Person;
- oder ein Merkmal einer Muskelaktivität der Person;
- oder ein Merkmal der kortikalen Aktivität der Person;
- oder ein Merkmal der elektrodermalen Aktivität der Person;
- oder eine Körpertemperatur der Person;
- oder ein Merkmal, das für eine Bewegung der Person repräsentativ ist.

8. Verfahren nach einem der vorhergehenden Ansprüche, wobei:

- die Schritte a) bis c) unabhängig voneinander jeweils für verschiedene physiologische Parameter ($x_i$) durchgeführt werden, um ebenso viele Zugehörigkeitsfunktionen ($f_i$) wie betrachtete physiologische Parameter zu ermitteln, wobei jede Zugehörigkeitsfunktion einem physiologischen Parameter zugeordnet wird;
- der Schritt d) ein Messen der verschiedenen physiologischen Parameter ($x_i(t)$) während der Messperiode umfasst und eine Bestimmung eines Stresspegels ($SI_i(t)$) der Person bezüglich jedes physiologischen Parameters mithilfe jeder Zugehörigkeitsfunktion ($f_i$), die jeweils jedem physiologischen Parameter zugeordnet ist;

wobei das Verfahren einen Schritt e) des Kombinierens der Stresspegel ($SI_i(t)$) umfasst, die bei dem Schritt d) bezüglich jedes physiologischen Parameters ($x_i$) bestimmt werden, um einen multiparametrischen Stresspegelindex ($SI(t)$) zu erhalten.

9. Verfahren nach Anspruch 8, wobei bei dem Schritt e) der multiparametrische Stresspegelindex durch eine Berechnung eines gewichteten Mittelwerts oder eines Medianwerts der Stresspegel ($Sl_i(t)$) bestimmt wird, die bezüglich jedes physiologischen Parameters ($x_i$) bestimmt werden.

10. Verfahren nach einem der Ansprüche 8 oder 9, wobei bei dem Schritt c) jede Zugehörigkeitsfunktion ($f_i$) unabhängig von den anderen definiert wird.

11. Verfahren nach einem der Ansprüche 2 bis 10, wobei die Zugehörigkeitsfunktion oder jede Zugehörigkeitsfunktion eine Funktion ist, die in einem Intervall definiert ist, das zwischen dem Variationsbereich und dem um den Schwellenabstand vergrößerten Variationsbereich liegt und in diesem Intervall stetig ist.

12. Vorrichtung zur Bestimmung eines Stresspegels einer Person, umfassend:

- einen Sensor (2), der dazu ausgestaltet ist, einen physiologischen Parameter der Person zu messen, wobei der Sensor einen Wert des Parameter misst, der in Abhängigkeit von dem Stresspegel der Person variieren kann;
- einen Prozessor (4), der dazu ausgestaltet ist, die Schritte b) bis c) eines Verfahrens nach einem der vorhergehenden Ansprüche ausgehend von Messungen von verschiedenen Werten des physiologischen Parameters durchzuführen, die von dem Sensor bei Kalibrierungsperioden gemessen werden, bei denen die Person als in Ruhe befindlich betrachtet wird, wobei der Prozessor auch dazu ausgestaltet ist, den Schritt d) ausgehend von mindestens einem Wert des physiologischen Parameters durchzuführen, der während einer Messperiode gemessen wird.

**Claims**

1. Method for determining a stress level of an individual, depending on a physiological parameter of the individual, the value of which is liable to vary depending on the stress level of the individual, the method comprising the following steps:

a) measuring a value of the physiological parameter ($x_r(t_r)$), in various calibration periods ($t_r$) using a sensor;
b) defining a range of variation ($X_r$) in the values of the physiological parameter which are measured in the various calibration periods;
c) depending on the range of variation ($X_r$), establishing a membership function ($f$), determining a stress level ($Sl$) of the individual depending on the value of the physiological parameter, the stress level determined by the membership function varying between:

- a rest level, corresponding to a rest state of the individual;
- a stress level, corresponding to a stressed state of the individual;
- and at least one intermediate level, comprised between the rest level and the stress level;

d) following step c), measuring a value of the physiological parameter ($x(t)$) in a measurement period, and determining the stress level of the individual ($Sl(t)$), during the measurement period, by applying the membership function ($f$) to the value of the physiological parameter measured during the measurement period;
the method being such that, in step c), the stress level and each intermediate level are determined:

- depending on values of the physiological parameters which are measured, in the calibration periods;
- and depending on a distance ($d(x(t), X_r)$) between the value of the physiological parameter measured in step d) and the range of variation ($X_r$) defined in step b);

the method being **characterized in that**, in step a), in each calibration period, the individual is considered to be in a rest state.

2. Method according to Claim 1, wherein step c) comprises taking into account a threshold distance ($d_s$), such that:

- the rest level corresponds to a value of the physiological parameter ($x(t)$) comprised in the range of variation ($X_r$) resulting from step b);
- the stress level corresponds to a value of the physiological parameter ($x(t)$) a distance ($d(x(t), X_r)$) of which, with respect to the range of variation ($X_r$), is larger than the threshold distance ($d_s$);

- each intermediate level corresponds to a value of the physiological parameter *(x(t))* outside of the range of variation *(X$_r$)*, and the distance *(d(x(t), X$_r$))* of which, with respect to the range of variation, is smaller than the threshold distance *(d$_s$)*.

3. Method according to Claim 2, wherein step c) comprises calculating an indicator of the dispersion of the values $(x_r(t_r))$ of the physiological parameter which are measured in the various calibration periods, the threshold distance $(d_s)$ being determined depending on the dispersion indicator.

4. Method according to Claim 3, wherein the dispersion indicator is or comprises an extent of the range of variation $(\Delta x_r)$, corresponding to a deviation between a minimum value $(x_{r,min})$ and a maximum value $(x_{r,max})$ of the range of variation.

5. Method according to Claim 4, wherein the threshold distance $(d_s)$ is obtained by applying a scale factor $(\alpha)$ to the extent of the range of variation.

6. Method according to any one of Claims 2 to 5, wherein step c) comprises attributing a value representative of the range of variation $(x_{r,max}, x_{r,min}, \overline{X}_r)$ according to which:

   - the stressed state corresponds to a value of the physiological parameter the distance of which, with respect to the representative value, is larger than the threshold distance $(d_s)$;
   - each intermediate level corresponds to a value of the physiological parameter the distance of which, with respect to the representative value, is smaller than the threshold distance $(d_s)$.

7. Method according to one of the preceding claims, wherein the physiological parameter is or comprises:

   - a feature of the cardiac activity of the individual;
   - or a feature of a muscular activity of the individual;
   - or a feature of the cortical activity of the individual;
   - or a feature of the electrodermal activity of the individual;
   - or a body temperature of the individual;
   - or a feature representative of a movement of the individual.

8. Method according to any one of the preceding claims, wherein:

   - steps a) to c) are implemented independently of one another, respectively for various physiological parameters $(x_i)$, in order to establish as many membership functions $(f_i)$ as physiological parameters in question, each membership function being associated with one physiological parameter;
   - step d) comprises measuring the various physiological parameters $(x_i(t))$, during the measurement period, and determining a stress level $(Sl_i(t))$ of the individual relative to each physiological parameter, using each membership function $(f_i)$ respectively associated with each physiological parameter;

   the method comprises a step e) of combining the stress levels $(Sl_i(t))$ determined relative to each physiological parameter $(x_i)$, in step d), in order to obtain a multi-feature stress-level index $(Sl(t))$.

9. Method according to Claim 8, wherein, in step e), the multi-feature stress-level index is determined by calculating a weighted mean or a median of the stress levels $(Sl_i(t))$ respectively determined relative to each physiological parameter $(x_i)$.

10. Method according to one of Claims 8 and 9, wherein, in step c), each membership function $(f_i)$ is defined independently of the others.

11. Method according to any one of Claims 2 to 10, wherein the membership function, or each membership function, is a function defined in an interval comprised between the range of variation and the range of variation increased by the threshold distance, and is continuous in this interval.

12. Device for determining a stress level of an individual, comprising:

- a sensor (2), configured to measure a physiological parameter of the individual, the sensor measuring a value of the parameter liable to vary depending on the stress level of the individual;
- a processor (4), configured to implement steps b) to c) of a method according to any of the preceding claims, on the basis of measurements of various values of the physiological parameter which are measured by the sensor in calibration periods, in which the individual is considered to be at rest, the processor also being configured to implement step d) on the basis of at least one value of the physiological parameter measured during a measurement period.

**Fig. 1**

**Fig. 2**

$Sl(t)$

1

$f$

$\Delta x_r$

0

$x_{r,min}$ $x_{r,max}$ $d_s$

$X_r$

$x(t)$

**Fig. 3A**

$Sl(t)$

1

$f$

$\Delta x_r$

0

$x_{r,min}$ $x_{r,max}$ $d_s$

$X_r$

$x(t)$

**Fig. 3B**

$Sl(t)$

1

$f$

7

0

78 85 7 92

$X_r$

$x(t)$

**Fig. 3C**

**Fig. 4**

D2  MST  SECPT  TSST

**Fig. 5A**

**Fig. 5B**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **OLLANDER.** A comparison of wearable and stationary sensors for stress détection. *2016 IEEE international conférence on Systems, Man and Cybernetics (SMC),* Octobre 2016

- **KUMAR M.** Fuzzy techniques for subjective workload-score modeling under uncertainties. *IEEE transactions on systems, man and cybernetics- part B,* Décembre 2008, vol. 38 (6
- **SANTOS SIERRA A.** Real-time stress détection by means of physiological signals. *Recent Application in Biometrics,* 27 Juillet 2011